(19) Europäisches Patentamt
European Patent Office
Office européen des brevets

(11) **EP 3 630 297 B1**

(12) **FASCICULE DE BREVET EUROPEEN**

(45) Date de publication et mention
de la délivrance du brevet:
**17.08.2022 Bulletin 2022/33**

(21) Numéro de dépôt: **18735649.8**

(22) Date de dépôt: **24.05.2018**

(51) Classification Internationale des Brevets (IPC):
**A61Q 19/00** (2006.01)  **A61K 8/92** (2006.01)
**A61K 8/04** (2006.01)

(52) Classification Coopérative des Brevets (CPC):
**A61K 8/046; A61K 8/92; A61K 8/922; A61Q 19/00;
A61Q 19/007;** A61K 2800/31

(86) Numéro de dépôt international:
**PCT/FR2018/051230**

(87) Numéro de publication internationale:
**WO 2018/220315 (06.12.2018 Gazette 2018/49)**

(54) **COMPOSITION COSMÉTIQUE FOISONNÉE À HAUTE TENEUR EN BEURRE VÉGÉTAL**

KOSMETISCHE SCHAUMZUSAMMENSETZUNG MIT HOHEM PFLANZENBUTTERGEHALT

COSMETIC FOAM COMPOSITION WITH A HIGH VEGETABLE BUTTER CONTENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.06.2017 FR 1754927**

(43) Date de publication de la demande:
**08.04.2020 Bulletin 2020/15**

(73) Titulaire: **LABORATOIRES M & L
04100 Manosque (FR)**

(72) Inventeurs:
• **ANDRIEU, Delphine
04700 Oraison (FR)**
• **LAUVERGEON, Marjorie
13770 Venelles (FR)**

(74) Mandataire: **Renard, Emmanuelle
Renard IP
4 Bis Avenue de Lorraine
92380 Garches (FR)**

(56) Documents cités:
**WO-A1-2004/000243     GB-A- 1 298 154
US-A1- 2006 147 390**

• **DATABASE GNPD [Online] MINTEL; 30 novembre
2016 (2016-11-30), "Body Cream", XP002775191,
Database accession no. 4395783**

EP 3 630 297 B1

**Description**

<u>OBJET DE L'INVENTION</u>

La présente invention concerne une composition cosmétique foisonnée, sensiblement anhydre, riche en beurre végétal, qui est stabilisée par au moins un tensioactif anionique choisi parmi les sels des acides aminés N-acylés par un acide gras. Elle se rapporte également au procédé de préparation de cette composition, ainsi qu'à son utilisation pour prévenir et/ou traiter les signes du dessèchement cutané.

<u>ARRIERE-PLAN DE L'INVENTION</u>

Le beurre de karité est un corps gras issu des fruits d'un arbre africain de l'espèce *Vitellaria paradoxa* (anciennement *Butyrospermum parkii* (G. Don) Kotschy), essentiellement constitué d'un mélange d'acide gras solides et liquides. Si le beurre de karité est très apprécié pour nourrir et protéger la peau et les lèvres, les compositions en contenant un taux élevé ont l'inconvénient de présenter une texture lourde et grasse qui peut détourner certains consommateurs de leur utilisation.

Pour remédier à cet inconvénient, il a déjà été suggéré de le formuler dans des compositions foisonnées, présentant une texture aérée. Ainsi, le document US 2006/0147390 décrit une composition anhydre foisonnée comprenant un mélange de lipides, telles que des huiles végétales, et de cires, dont la fraction solide du beurre de karité. Les lipides représentent avantageusement de 75 à 85% du poids de la composition. Il est indiqué que ces compositions sont stables en l'absence d'émulsifiant. La Demanderesse a toutefois démontré que l'utilisation d'un ratio cires / huiles plus élevé que ceux suggérés dans ce document entraînait une déstabilisation de la composition.

Des solutions ont été proposées pour remédier à ce problème de stabilité. Notamment, le document US-8,802,118 décrit des compositions foisonnées pour les lèvres, comprenant essentiellement des corps gras, à savoir une cire, une substance pâteuse et une huile non volatile. La substance pâteuse peut être du beurre de karité, représentant jusqu'à 50% du poids de la composition. Les problèmes de stabilité précités sont résolus par l'incorporation de silice. D'autres compositions foisonnées à phase grasse continue ont été proposées dans les documents FR 2 915 892 et FR 2 915 877. Ces compositions sont stabilisées par incorporation d'un polymère structurant synthétique ou de palmitate de dextrine, respectivement.

Une autre solution pour obtenir une composition foisonnée stable, riche en corps gras, a été proposée dans le document WO 2016/150978. Elle consiste à refroidir la composition à -23°C pour faire cristalliser les lipides, puis à ramener sa température à environ 5°C avant d'y introduire de l'air. Les proportions de lipides sont par ailleurs ajustées de telle manière que les huiles sont présentes en plus grande quantité que les cires et les beurres.

Après de nombreuses recherches, il est apparu à la Demanderesse que des taux plus élevés de beurre de karité pouvaient être formulés dans une composition foisonnée que ce qui a été proposé dans l'art antérieur, sans entraîner de problèmes de stabilité et sans avoir recours pour autant aux stabilisants mentionnés précédemment ni à un procédé de fabrication complexe, par incorporation dans la composition d'un tensioactif anionique sous forme de sel d'acide aminé acylé.

<u>RESUME DE L'INVENTION</u>

L'invention a ainsi pour objet une composition cosmétique comprenant, dans un milieu physiologiquement acceptable :

- de 30 à 90% en poids d'au moins un beurre végétal,
- au moins un tensioactif anionique choisi parmi les sels des acides aminés N-acylés par un acide gras en $C_6$-$C_{20}$, de préférence en $C_{12}$-$C_{18}$,
- au moins une cire,
- au moins une huile,
- de 0 à 5% en poids d'eau, et
- au moins un gaz,

les pourcentages ci-dessus étant exprimés par rapport au poids total de la composition, la composition ayant une densité comprise entre 0,40 et 0,75 g/cm$^3$.

Elle a également pour objet un procédé de préparation de cette composition, comprenant les étapes successives suivantes :

- le mélange du beurre végétal, de la cire et de l'huile, à une température de 70 à 80°C,

- le refroidissement du mélange à une température de 25 à 40°C,
- l'introduction d'air dans ledit mélange au moyen d'un mélangeur, jusqu'à atteindre un taux de foisonnement de 30 à 150%, et
- la maturation de la composition foisonnée ainsi obtenue.

Elle a encore pour objet l'utilisation cosmétique de cette composition pour prévenir ou traiter les signes du dessèchement cutané, notamment pour nourrir et/ou assouplir la peau.

DESCRIPTION DETAILLEE

Comme indiqué précédemment, la composition selon l'invention comprend un mélange de corps gras et un tensioactif particulier, dans un milieu physiologiquement acceptable. Ces constituants seront à présent décrits plus en détail.

Par « milieu physiologiquement acceptable », on entend en particulier un milieu cosmétiquement acceptable, c'est-à-dire qui ne génère pas de picotements ou de rougeurs incompatibles avec une utilisation cosmétique. Ce milieu est constitué d'une phase grasse continue, la composition contenant précisément de 0 à 5% en poids d'eau, de préférence de 0 à 3%, voire de 0 à 2% en poids d'eau. Il s'agit donc d'une composition anhydre, c'est-à-dire préparée en l'absence d'ajout d'eau autre que celle qui peut être intrinsèquement présente dans les matières premières utilisées.

Corps gras

La composition cosmétique selon l'invention comprend un taux élevé de beurre végétal, c'est-à-dire de 30 à 90% en poids de beurre, par exemple de 50 à 80% en poids, voire de 60 à 80% en poids, de beurre et préférentiellement de 40 à 75% en poids, par exemple de 50 à 75% en poids et, mieux, de 60 à 70% en poids, de beurre.

Par « beurre végétal », on entend un corps gras pâteux à changement d'état liquide / solide réversible, présentant à l'état solide une organisation cristalline anisotrope et comportant à une température de 23°C une fraction liquide et une fraction solide. Des exemples de beurres végétaux sont les beurres de karité, de cacao et de mangue, et leurs mélanges. On préfère utiliser le beurre de karité.

Outre le beurre végétal, la composition selon l'invention comprend une ou plusieurs huiles volatiles et/ou non volatiles. Dans le contexte de cette description, on entend par "huile" un composé liquide à température ambiante (25°C) et pression atmosphérique ($10^5$ Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau. Par "huile volatile", on entend une huile ayant une pression de vapeur de l'ordre de 0,001 à 300 mm Hg et de préférence de 0,01 à 10 mm Hg à température ambiante (20°C) et pression atmosphérique. Avantageusement, ces huiles présentent en outre un point éclair (mesuré selon la norme ASTM D93) inférieur à 100°C, de préférence compris entre 50 et 95°C, par exemple entre 70 et 90°C, voire entre 80 et 90°C et/ou une viscosité cinématique inférieure à 5 cSt, voire comprise entre 1 et 3 cSt, à 40°C.

Des exemples d'huiles volatiles sont les alcanes ramifiés, tels que l'isododécane, et les alcanes linéaires en $C_{10}$-$C_{13}$.

Comme huiles non volatiles, on peut citer notamment :

- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le caprylate de propylheptyle et leurs mélanges. D'autres esters utilisables sont les diesters d'acides dicarboxyliques et de monoalcools tels que l'adipate de diisopropyle, l'adipate de diéthylhexyle, le sébaçate de diisopropyle et le sébaçate de diisoamyle.

On peut également citer les huiles végétales qui contiennent un ou plusieurs des constituants précités.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

On préfère selon l'invention utiliser des huiles non volatiles et plus préférentiellement des huiles végétales. Les huiles peuvent représenter de 10 à 40% et de préférence de 15 à 25% du poids total de la composition.

La composition selon l'invention comprend par ailleurs au moins une cire. Le terme "cire" désigne, dans le contexte de cette description, un corps gras solide à 25°C, à changement d'état solide / liquide réversible, ayant un point de fusion généralement compris entre 30 et 160°C, de préférence entre 50 et 90°C, tel que mesuré par Calorimétrie à Balayage Différentiel (DSC). Des exemples de cires sont en particulier les cires d'origine animale ou végétale, telles que les cires d'abeille, de candelilla, de carnauba ou d'acacia ; les esters d'acides gras linéaires saturés en $C_{14}$-$C_{30}$ et d'alcools gras linéaires saturés en $C_{16}$-$C_{36}$ ; les acides linéaires et saturés en $C_{10}$-$C_{30}$ ; les alcools linéaires et saturés en $C_8$-$C_{30}$ ; et leurs mélanges. On peut également citer le mélange de triglycérides végétaux, ayant un point de fusion glissant de 45-55°C, commercialisé par la société UNIVAR sous la dénomination commerciale Cremerlin® Pura. Ces cires peuvent se trouver sous forme micronisée, c'est-à-dire sous la forme d'une poudre dont les particules présentent une taille moyenne en nombre inférieure ou égale à 50 $\mu$m, et notamment allant de 0,5 à 50 $\mu$m, de préférence allant de 1 à 30 $\mu$m, voire allant de 3 à 20 $\mu$m, où la « taille moyenne en nombre» correspond à la dimension donnée par la distribution granulométrique statistique à la moitié de la population, dite D50.

Selon une forme d'exécution préférée, la cire est choisie parmi les huiles végétales hydrogénées, éventuellement associée à une autre cire telle qu'une cire de triglycérides végétaux. L'huile végétale hydrogénée peut en particulier être choisie parmi les huiles de colza, de soja, de tournesol, de jojoba, de coco, de coprah et de ricin hydrogénées, et leurs mélanges. On utilise de préférence l'huile de ricin hydrogénée.

Selon un mode de réalisation préféré de l'invention, la composition selon l'invention ne renferme pas d'autre cire que des cires d'origine végétale.

Les cires peuvent représenter de 0,5 à 5% du poids total de la composition.

On préfère en outre qu'hormis les constituants précités, cette composition ne contienne pas de gélifiant(s) de phase grasse. Par "gélifiants de phase grasse", il est fait référence aux composés modifiant la rhéologie de la phase grasse par formation d'un réseau tridimensionnel. Comme composés de ce type, on peut notamment citer les argiles (en particulier les bentonites et les hectorites) modifiées hydrophobes, notamment par du chlorure de di-stéaryl di-méthyl ammonium ; les silices pyrogénées modifiées hydrophobes ; le palmitate et le myristate de dextrine ; les polyamides, les copolymères oléfine(s) / styrène, les poly(acrylates d'alkyle) ; les glycérides d'acides gras (de préférence linéaires et saturés) en $C_{16}$-$C_{26}$ tels que le composé Nomcort® HKG ; les dérivés de cellulose et les mélanges en contenant ; et leurs mélanges.

Tensioactif anionique

La composition selon l'invention renferme un tensioactif anionique particulier, choisi parmi les sels des acides aminés N-acylés par un acide gras en $C_6$-$C_{20}$, de préférence en $C_{12}$-$C_{18}$. Il a en effet été démontré que ce tensioactif permettait l'obtention d'une formule stable avant et après foisonnement et présentant de bonnes propriétés cosmétiques, notamment une texture souple et s'étalant facilement. La stabilité de la composition peut notamment se traduire par l'absence d'exsudation et/ou d'affaissement de la mousse, en particulier lorsque la composition est conservée pendant une semaine à 60°C et/ou deux mois à 50°C et/ou six mois à 40°C.

Ce tensioactif anionique peut avantageusement être choisi parmi : les sels inorganiques, notamment de sodium ou de potassium, de l'acide glutamique acylé et des hydrolysats de protéines végétales acylés.

Des exemples de tels tensioactifs anioniques peuvent être choisis parmi : le sel de sodium du lauroyl glutamate, les sels de sodium et de potassium du cocoyl glutamate, le sel disodique du capryloyl glutamate, le sel de magnésium du palmitoyl glutamate, le sel de potassium du myristoyl glutamate, le sel de sodium du lauroyl aspartate, le sel de sodium du lauroyl glycinate, le sel de sodium et de potassium du cocoyl glycinate, le sel de sodium du lauroyl sarcosinate, le sel de sodium du cocoyl alaninate, les sels de sodium et de potassium des hydrolysats de protéines d'avoine ou de blé acylés par l'acide laurique, et leurs mélanges. On préfère selon l'invention utiliser le sel de sodium du lauroyl glutamate.

Le tensioactif anionique peut représenter de 0,2 à 2% en poids, et de préférence de 0,5 à 1% en poids, par rapport au poids total de la composition.

Gaz

La composition selon l'invention renferme au moins un gaz, qui peut notamment être choisi parmi l'air, l'azote, l'oxygène et le dioxyde de carbone, ainsi que leurs mélanges. L'air et l'azote sont préférés pour une utilisation dans la présente invention.

La composition peut ainsi être assimilée à une émulsion gaz-dans-lipides présentant l'aspect d'une crème semi-solide dans laquelle sont piégées des bulles de gaz présentant par exemple une taille de 0,5 à 50 $\mu$m. Contrairement aux compositions distribuées dans un aérosol, dans lesquelles la mousse se forme uniquement au moment de l'utilisation, la composition selon l'invention peut être conditionnée sous forme de mousse et stockée sous cette forme pendant plusieurs mois dans son conditionnement. Dans une forme d'exécution préférée de l'invention, la composition selon l'invention est conditionnée dans un pot.

En raison de la présence du gaz précité au sein de sa structure, la composition présente une densité comprise entre 0,40 et 0,75 g/cm$^3$ et de préférence entre 0,45 et 0,65 g/cm$^3$. La densité peut être mesurée de manière classique en utilisant un pycnomètre ou bécher de volume connu.

Le taux de foisonnement $\Phi$ de la composition est défini comme l'augmentation du volume de composition dû à l'incorporation de la phase gaz et illustré par la formule suivante :

$$\Phi = [(\rho_c - \rho_m) / \rho_m] * 100$$

où $\rho_c$ est la densité de la composition avant foisonnement et $\rho_m$ est sa densité après foisonnement. Ce taux de foisonnement est généralement compris entre 30 et 150% et de préférence entre 50 et 110%, notamment entre 60 et 80%.

Autres constituants

La composition utilisée selon l'invention peut par ailleurs comprendre un ou plusieurs autres constituants choisis par exemple parmi les émulsionnants, parfums, conservateurs, séquestrants, anti-oxydants, ajusteurs de pH, filtres UV, charges pulvérulentes, pigments, colorants et leurs mélanges.

Selon une forme d'exécution préférée, cette composition renferme au moins un autre tensioactif anionique qui est un sel d'acyl lactylate dont le groupement acyle renferme de 14 à 22 atomes de carbone, de préférence le sel de sodium du lauroyl lactylate ou du stéaroyl lactylate. ce tensioactif additionnel peut représenter de 0,2 à 2% et de préférence de 0,1 à 1% du poids total de la composition.

En variante ou en plus, la composition peut en outre contenir au moins un tensioactif non ionique, avantageusement constitué d'un ester d'acide gras de glycérol ou de polyglycérol éventuellement associé à au moins un alcool gras comme co-tensioactif. Le tensioactif non ionique peut représenter de 1 à 10% et de préférence de 2 à 6% du poids total de la composition.

Selon un mode de réalisation préféré, la composition selon l'invention renferme en outre au moins une charge pulvérulente, destinée à améliorer son toucher, qui peut se présenter sous forme de microparticules poreuses ou creuses, de préférence poreuses. Ces microparticules sont en principe sensiblement sphériques. Ces charges peuvent notamment être choisies parmi :

- les charges organiques telles que : les poudres de polysaccharides et en particulier d'amidon natif, d'amidon modifié ou de cellulose ; les poudres de polymères acryliques tels que le poly(méthacrylate de méthyle), de polyamides ou de polyoléfines ; les poudres d'algues séchées telles que *Corallina officinalis* ;
- les charges inorganiques telles que la silice, les argiles, la perlite et le talc ;
- et leurs mélanges.

Les amidons sont de préférence choisis parmi l'amidon de maïs, de riz, de tapioca ou de blé. Des exemples d'amidons modifiés sont les amidons éventuellement pré-gélatinisés et/ou oxydés, qui sont estérifiés par un anhydride alkénylsuccinique, notamment par l'anhydride octénylsuccinique ou dodécylsuccinique, éventuellement en présence de chlorure de calcium, ainsi que les amidons éthérifiés, notamment hydroxypropylés ou carboxyméthylés, et les amidons cationiques, notamment quaternisés. On peut également citer l'amidon réticulé par le trimétaphosphate de sodium. Les amidons, de préférence natifs, constituent des charges organiques préférées pour une utilisation dans cette invention.

Ces charges peuvent représenter de 1 à 10% en poids, et de préférence de 2 à 5% en poids, par rapport au poids total de la composition.

La composition peut également comprendre un ou plusieurs actifs hydratants et notamment des polyols, tels que de la glycérine ou du propylène glycol, représentant par exemple de 0,1 à 5% et de préférence de 0,3 à 1% du poids total de la composition.

Procédé de préparation

La composition décrite précédemment peut être préparée suivant un procédé comprenant les étapes successives suivantes :

- le mélange du beurre végétal, de la cire et de l'huile, à une température de 70 à 80°C,
- le refroidissement du mélange à une température de 25 à 40°C,
- l'introduction de gaz dans ledit mélange jusqu'à atteindre un taux de foisonnement de 30 à 150%, de préférence de 50 à 110%, et
- la maturation de la composition foisonnée ainsi obtenue, par exemple pendant une durée d'au moins 24h, de préférence d'au moins 3 jours, mieux, d'au moins 6 jours.

L'introduction de gaz dans la composition est réalisée dans un mélangeur, comprenant typiquement des moyens d'agitation mécanique et des moyens d'amenée de gaz. Des mélangeurs de ce type sont notamment les systèmes d'aération disponibles auprès de la société HAAS sous la dénomination commerciale Mondomix®. Il peut être avantageux de prévoir une étape de maturation de la composition avant foisonnement, pendant une durée d'au moins 24h, par exemple d'au moins 2 jours, voire d'au moins 3 jours, voire encore d'au moins 6 jours.

Utilisation

La composition selon l'invention se présente généralement sous forme de crème épaisse ayant la texture d'un beurre. Il s'agit généralement d'une composition non rincée.
Elle peut être utilisée, par application topique sur la peau, pour prévenir ou traiter les signes du dessèchement cutané, notamment pour nourrir et/ou assouplir la peau. En variante, elle peut être utilisée pour nourrir les cheveux, en particulier les pointes. La composition selon l'invention peut ainsi être appliquée sur toutes zones du corps et des cheveux chez l'adulte et l'enfant, plus particulièrement sur le visage, le cou et/ou le décolleté, ou sur les zones plus sèches telles que les coudes, les genoux, les pieds, les cheveux et/ou les lèvres. Elle peut être appliquée à raison d'une à deux fois par jour, de préférence matin et/ou soir.

EXEMPLES

L'invention sera mieux comprise à la lumière des exemples suivants, qui sont donnés à titre purement illustratif et n'ont pas pour but de limiter la portée de l'invention, définie par les revendications annexées.

**Exemple 1** : **Préparation d'une composition selon l'invention**

On a préparé un beurre fouetté à partir des constituants indiqués ci-dessous, utilisés dans les proportions suivantes :

| | |
|---|---|
| Beurre de karité | 70,00 % |
| Cire | 2,00 % |
| Huiles végétales | qsp 100 % |
| Huile de ricin hydrogénée | 1,5 % |
| Tensioactifs non ioniques | 3,2 % |
| Sel de sodium du lauroyl glutamate | 0,4 % |
| Sel de sodium du stéaroyl lactylate | 0,4 % |
| Anti-oxydant | qs |
| Amidon de tapioca | 5,00 % |
| Base parfumée | qs |
| Eau | < 1% |

Pour ce faire, on a fait fondre les corps gras et les conservateurs à 75°C, puis on a ajouté l'anti-oxydant à ce mélange qui a ensuite été homogénéisé sous agitation à 500 tours/min. Après refroidissement à 50°C, on a ajouté la charge pulvérulente sous agitation, puis l'ensemble a été refroidi à 30°C, la base parfumée étant ajoutée pendant cette étape de refroidissement.

La composition ainsi obtenue a été étuvée entre 25 et 40°C puis transférée dans un dispositif de type Mondomix® (HAAS) pourvu d'une pompe d'alimentation, d'une tête de mélange permettant de cisailler la composition et d'y introduire un gaz et d'une vanne de contre-pression. Les paramètres de l'appareil ont été réglés pour obtenir un taux de foisonnement compris entre 30 et 150%.

Le beurre obtenu présentait un aspect aéré, ressemblant à une crème battue de couleur blanche qui possédait une structure souple et craquante lors de sa prise.

**Exemple 2** : **Tests de stabilité**

On a comparé la stabilité à 50°C avant foisonnement d'une composition similaire à celle de l'Exemple 1 (ci-après « C1 ») avec des compositions qui en différaient essentiellement par la présence de tensioactifs anioniques similaires (ci-après « C2 », « C3 » et « C5 ») ou par l'absence de tensioactif anionique (« C4 »). Des essais complémentaires ont été réalisés pour comparer la stabilité de la composition de l'Exemple 1 avant foisonnement (formule A1) avec deux compositions identiques (formules A2 et A3), excepté qu'elles contenaient uniquement des tensioactifs non ioniques, le pourcentage total de tensioactifs étant inchangé. Ces trois compositions ont été conservées pendant une semaine à 60°C.

Les résultats obtenus sont rassemblés dans le Tableau 1 ci-après.

Comme il ressort de ce tableau, les compositions C1, C2, C3 et C5 (respectivement A1) selon l'invention sont plus stables que la composition C4 (respectivement, A2 ou A3) qui ne renferme pas de tensioactif anionique sous forme de sel d'acide(s) aminé(s) acylé(s).

Tableau 1

| | C1 | C2 | C3 | C4 | C5 | A1 | A2 | A3 |
|---|---|---|---|---|---|---|---|---|
| Beurre(s) | 70% | 70% | 70% | 70% | 72% | 70% | 70% | 70% |
| Huiles végétales | qsp 100% | qsp 100% | qsp 100% | qsp 100% | qsp 100% | qsp 100% | qsp 100% | qsp 100% |
| Huile de ricin hydrogénée | 0,5% | 0,5% | 1% | 1% | 0,6% | 1,5% | 1,5% | 1,5% |
| Cire de triglycérides | 2% | 2% | 2% | 2% | -- | 2% | 2% | 2% |
| Lauroyl glutamate de sodium | 0,6%[5] | -- | 0,8%[3] | -- | -- | 0,4%[5] | -- | -- |
| Sel de potassium d'hydrolysat de protéine de riz acylée | -- | 0,45%[2] | -- | -- | 0,45%[2] | -- | -- | -- |
| Sel de sodium d'acides aminés acylés | -- | 0,45%[2] | -- | -- | 0,45%[2] | -- | -- | -- |
| Charge pulvérulente | 3% | 3% | 3% | 6% | 3% | 5% | 5% | 5% |
| Sel de sodium du stéaroyl lactylate | 0,6%[5] | -- | -- | -- | -- | 0,4%[5] | -- | -- |
| Protéine végétale hydrolysée | -- | | -- | -- | -- | -- | -- | -- |
| Tensioactifs non ioniques | 4,8%[5] | -- | 2,3%[3] | 3%[4] | -- | 3,2%[5] | 4,0%[6] | 4,0%[7] |

(suite)

| | C1 | C2 | C3 | C4 | C5 | A1 | A2 | A3 |
|---|---|---|---|---|---|---|---|---|
| Glycérine | -- | 0,4%(2) | -- | -- | 0,4%(2) | -- | -- | -- |
| Base parfumée | qs | qs | qs | qs | qs | qs | qs | qs |
| Observations | Stable | Stable | Stable | Déphase à 50°C après une semaine | Stable | Stable | Exsude en surface | Croûte blanchâtre en surface |

(1) Olivoil Avenate® Emulsifier de KALICHEM ; (2) Avogelia® de VARIATI ; (3) Protelan® ENS de ZSCHIMMER & SCHWARZ ; (4) S-FACE® VL 212 de SAKAMOTO YAKUHIN KOGYO ; (5) Protelan® NMF de ZSCHIMMER & SCHWARZ ; (6) GLYCERYL STEARATE ; (7) Alcool cétéarylique

## Revendications

1. Composition cosmétique comprenant, dans un milieu physiologiquement acceptable :

   - de 30 à 90% en poids d'au moins un beurre végétal,
   - au moins un tensioactif anionique choisi parmi les sels des acides aminés N-acylés par un acide gras en $C_6$-$C_{20}$,
   - au moins une cire,
   - au moins une huile,
   - de 0 à 5% en poids d'eau, et
   - au moins un gaz,

   les pourcentages ci-dessus étant exprimés par rapport au poids total de la composition, la composition ayant une densité comprise entre 0,40 et 0,75 g/cm$^3$.

2. Composition selon la revendication 1, **caractérisée en ce que** le tensioactif anionique est choisi parmi : les sels inorganiques, notamment de sodium ou de potassium, de l'acide glutamique acylé et des hydrolysats de protéines végétales acylés, et leurs mélanges.

3. Composition selon la revendication 2, **caractérisée en ce que** le tensioactif anionique est choisi parmi : le sel de sodium du lauroyl glutamate, les sels de sodium et de potassium du cocoyl glutamate, le sel disodique du capryloyl glutamate, le sel de magnésium du palmitoyl glutamate, le sel de potassium du myristoyl glutamate, le sel de sodium du lauroyl aspartate, le sel de sodium du lauroyl glycinate, les sels de sodium et de potassium du cocoyl glycinate, le sel de sodium du lauroyl sarcosinate, le sel de sodium du cocoyl alaninate, les sels de sodium et de potassium des hydrolysats de protéines d'avoine ou de blé acylés par l'acide laurique, de préférence le sel de sodium du lauroyl glutamate.

4. Composition selon l'une quelconque des revendications 1 à 3, **caractérisée en ce que** le tensioactif anionique représente de 0,2 à 2% en poids, par rapport au poids total de la composition.

5. Composition selon l'une quelconque des revendications 1 à 4, **caractérisée en ce qu'**elle renferme en outre au moins un tensioactif non ionique, avantageusement constitué d'un ester d'acide gras de glycérol ou de polyglycérol éventuellement associé à au moins un alcool gras.

6. Composition selon l'une quelconque des revendications 1 à 5, **caractérisée en ce qu'**elle renferme en outre au moins une charge pulvérulente choisie parmi : les charges organiques telles que les poudres de polysaccharides et en particulier d'amidon natif, d'amidon modifié et de cellulose ; les poudres de polymères acryliques tels que le poly(méthacrylate de méthyle), de polyamides ou de polyoléfines ; les poudres d'algues séchées telles que *Corallina officinalis* ; les charges inorganiques telles que la silice, les argiles, la perlite et le talc ; et leurs mélanges ; de préférence un amidon.

7. Composition selon l'une quelconque des revendications 1 à 6, **caractérisée en ce qu'**elle renferme en outre au moins un sel d'acyl lactylate dont le groupement acyle renferme de 14 à 22 atomes de carbone, de préférence le sel de sodium du lauroyl lactylate ou du stéaroyl lactylate.

8. Composition selon l'une quelconque des revendications 1 à 7, **caractérisée en ce que** le beurre végétal est choisi parmi les beurres de karité, de cacao et de mangue, et leurs mélanges, de préférence le beurre végétal est le beurre de karité.

9. Composition selon l'une quelconque des revendications 1 à 8, **caractérisée en ce que** le beurre végétal représente de 40 à 75% en poids, par rapport au poids total de la composition.

10. Composition selon l'une quelconque des revendications 1 à 9, **caractérisée en ce que** la cire est choisie parmi les huiles végétales hydrogénées éventuellement associée à une autre cire et **en ce qu'**elle représente de préférence de 0,5 à 5% en poids, par rapport au poids total de la composition.

11. Composition selon l'une quelconque des revendications 1 à 10, **caractérisée en ce que** l'huile représente de 10 à 40% en poids, par rapport au poids total de la composition.

12. Composition selon l'une quelconque des revendications 1 à 11, **caractérisée en ce qu'**elle est conditionnée dans un pot.

13. Procédé de préparation de la composition selon l'une quelconque des revendications 1 à 11, **caractérisé en ce qu'**il comprend les étapes successives suivantes :

   - le mélange du beurre végétal, de la cire et de l'huile, à une température de 70 à 80°C,
   - le refroidissement du mélange à une température de 25 à 40°C,
   - l'introduction d'air dans ledit mélange jusqu'à atteindre un taux de foisonnement de 30 à 150%, le taux de foisonnement étant défini et mesuré conformément à la description, et
   - la maturation de la composition foisonnée ainsi obtenue.

14. Utilisation cosmétique de la composition selon l'une quelconque des revendications 1 à 11, pour prévenir et/ou traiter les signes du dessèchement cutané, notamment pour nourrir et/ou assouplir la peau.

**Patentansprüche**

1. Kosmetische Zusammensetzung, die in einem physiologisch annehmbaren Medium Folgendes umfasst:

   - 30 bis 90 Gew.-% mindestens einer Pflanzenbutter,
   - mindestens ein anionisches Tensid, das aus den Salzen von durch eine $C_6$-$C_{20}$-Fettsäure N-acylierten Aminosäuren ausgewählt ist,
   - mindestens ein Wachs,
   - mindestens ein Öl,
   - 0 bis 5 Gew.-% Wasser und
   - mindestens ein Gas,

   wobei die vorstehenden Prozentwerte bezogen auf das Gesamtgewicht der Zusammensetzung ausgedrückt sind, wobei die Zusammensetzung eine Dichte zwischen 0,40 und 0,75 g/cm$^3$ aufweist.

2. Zusammensetzung nach Anspruch 1, **dadurch gekennzeichnet, dass** das anionische Tensid aus anorganischen Salzen, insbesondere den Natrium- oder Kaliumsalzen von acylierter Glutaminsäure und acylierten pflanzlichen Proteinhydrolysaten, und Mischungen davon ausgewählt ist.

3. Zusammensetzung nach Anspruch 2, **dadurch gekennzeichnet, dass** das anionische Tensid aus dem Natriumsalz von Lauroylglutamat, den Natrium- und Kaliumsalzen von Cocoylglutamat, dem Dinatriumsalz von Capryloylglutamat, dem Magnesiumsalz von Palmitoylglutamat, dem Kaliumsalz von Myristoylglutamat, dem Natriumsalz von Lauroylaspartat, dem Natriumsalz von Lauroylglycinat, den Natrium- und Kaliumsalzen von Cocoylglycinat, dem Natriumsalz von Lauroylsarcosinat, dem Natriumsalz von Cocoylalaninat, den Natrium- und Kaliumsalzen von durch Laurinsäure acylierten Hafer- oder Weizenproteinhydrolysaten, vorzugsweise aus dem Natriumsalz von Lauroylglutamat, ausgewählt ist.

**4.** Zusammensetzung nach einem der Ansprüche 1 bis 3, **dadurch gekennzeichnet, dass** das anionische Tensid 0,2 bis 2 Gew.-%, bezogen auf das Gesamtgewicht der Zusammensetzung, darstellt.

**5.** Zusammensetzung nach einem der Ansprüche 1 bis 4, **dadurch gekennzeichnet, dass** sie außerdem mindestens ein nichtionisches Tensid enthält, das vorteilhafterweise aus einem Glycerin- oder Polyglycerinfettsäureester besteht, der gegebenenfalls mit mindestens einem Fettalkohol verbunden ist.

**6.** Zusammensetzung nach einem der Ansprüche 1 bis 5, **dadurch gekennzeichnet, dass** sie außerdem mindestens ein pulverförmiges Füllmittel enthält, das aus organischen Füllmitteln wie Polysaccharidpulvern und insbesondere natürlicher Stärke, modifizierter Stärke und Cellulose; Pulvern von Acrylpolymeren wie Poly(methylmethacrylat), Polyamiden oder Polyolefinen; Pulvern von getrockneten Algen wie *Corallina officinalis*; anorganischen Füllmitteln wie Siliciumdioxid, Tonen, Perlit und Talk und deren Mischungen, vorzugsweise einer Stärke, ausgewählt ist.

**7.** Zusammensetzung nach einem der Ansprüche 1 bis 6, **dadurch gekennzeichnet, dass** sie außerdem mindestens ein Salz eines Acyllactylats, dessen Acylgruppe 14 bis 22 Kohlenstoffatome enthält, vorzugsweise das Natriumsalz von Lauroyllactylat oder Steaoryllactylat, enthält.

**8.** Zusammensetzung nach einem der Ansprüche 1 bis 7, **dadurch gekennzeichnet, dass** die Pflanzenbutter aus Karite-, Kakao- und Mangobutter und ihren Mischungen ausgewählt ist, wobei es sich bei der Pflanzenbutter vorzugsweise um Karite-Butter handelt.

**9.** Zusammensetzung nach einem der Ansprüche 1 bis 8, **dadurch gekennzeichnet, dass** die Pflanzenbutter 40 bis 75 Gew.-%, bezogen auf das Gesamtgewicht der Zusammensetzung, darstellt.

**10.** Zusammensetzung nach einem der Ansprüche 1 bis 9, **dadurch gekennzeichnet, dass** das Wachs aus hydrierten Pflanzenölen ausgewählt ist, die gegebenenfalls mit einem anderen Wachs verbunden sind, und dadurch, dass es vorzugsweise 0,5 bis 5 Gew.-%, bezogen auf das Gesamtgewicht der Zusammensetzung, darstellt.

**11.** Zusammensetzung nach einem der Ansprüche 1 bis 10, **dadurch gekennzeichnet, dass** das Öl 10 bis 40 Gew.-%, bezogen auf das Gesamtgewicht der Zusammensetzung, darstellt.

**12.** Zusammensetzung nach einem der Ansprüche 1 bis 11, **dadurch gekennzeichnet, dass** sie in einem Behälter konditioniert wird.

**13.** Verfahren zur Herstellung der Zusammensetzung nach einem Ansprüche 1 bis 11, **dadurch gekennzeichnet, dass** es die folgenden aufeinanderfolgenden Schritte umfasst:

- die Mischung der Pflanzenbutter, Wachs und Öl auf eine Temperatur von 70 bis 80 °C,
- das Abkühlen der Mischung auf eine Temperatur von 25 bis 40 °C,
- das Einführen von Luft in die Mischung, bis ein Schäumungsgrad von 30 bis 150 % erreicht ist, wobei der Schäumungsgrad gemäß der Beschreibung definiert und gemessen wird, und
- das Reifen der so erhaltenen geschäumten Zusammensetzung.

**14.** Kosmetische Verwendung der Zusammensetzung nach einem der Ansprüche 1 bis 11 zur Prävention und/oder Behandlung von Symptomen der Austrocknung der Haut, insbesondere, um die Haut zu nähren und/oder zu geschmeidig zu machen.

**Claims**

**1.** A cosmetic composition comprising, in a physiologically acceptable medium:

- from 30 to 90% by weight of at least one vegetable butter,
- at least one anionic surfactant selected from the salts of amino acids N-acylated with a $C_6$-$C_{20}$ fatty acid,
- at least one wax,
- at least one oil,
- from 0 to 5% by weight of water, and
- at least one gas,

the above percentages being expressed in relation to the total weight of the composition, the composition having a density between 0.40 and 0.75 g/cm$^3$.

2. The composition as claimed in claim 1, **characterized in that** the anionic surfactant is selected from: inorganic salts, in particular sodium or potassium salts, of acylated glutamic acid and of acylated vegetable protein hydrolyzates, and mixtures thereof.

3. The composition as claimed in claim 2, **characterized in that** the anionic surfactant is selected from: the sodium salt of lauroyl glutamate, the sodium and potassium salts of cocoyl glutamate, the disodium salt of capryloyl glutamate, the magnesium salt of palmitoyl glutamate, the potassium salt of myristoyl glutamate, the sodium salt of lauroyl aspartate, the sodium salt of lauroyl glycinate, the sodium and potassium salts of cocoyl glycinate, the sodium salt of lauroyl sarcosinate, the sodium salt of cocoyl alaninate, the sodium and potassium salts of oat or wheat protein hydrolyzates acylated with lauric acid, preferably sodium salt of lauroyl glutamate.

4. The composition as claimed in any one of claims 1 to 3, **characterized in that** the anionic surfactant represents from 0.2 to 2% by weight, based on the total weight of the composition.

5. The composition as claimed in any one of claims 1 to 4, **characterized in that** it further contains at least one non-ionic surfactant, advantageously composed of a glycerol or polyglycerol fatty acid ester optionally combined with at least one fatty alcohol.

6. The composition as claimed in any one of claims 1 to 5, **characterized in that** it further contains at least one pulverulent filler selected from: organic fillers such as polysaccharide powders and in particular native starch, modified starch and cellulose; acrylic polymer powders such as poly(methyl methacrylate), polyamides or polyolefins; dried seaweed powders such as *Corallina officinalis*; inorganic fillers such as silica, clays, perlite and talc; and mixtures thereof; preferably a starch.

7. The composition as claimed in any one of claims 1 to 6, **characterized in that** it further contains at least one acyl lactylate salt whose acyl group contains from 14 to 22 carbon atoms, preferably the sodium salt of lauroyl lactylate or of stearoyl lactylate.

8. The composition as claimed in any one of claims 1 to 7, **characterized in that** the vegetable butter is selected from shea, cocoa and mango butters, and mixtures thereof, preferably the vegetable butter is shea butter.

9. The composition as claimed in any one of claims 1 to 8, **characterized in that** the vegetable butter represents 40 to 75% by weight, based on the total weight of the composition.

10. The composition as claimed in any one of claims 1 to 9, **characterized in that** the wax is selected from hydrogenated vegetable oils optionally combined with another wax and **in that** it preferably represents 0.5 to 5% by weight, based on the total weight of the composition.

11. The composition as claimed in any one of claims 1 to 10, **characterized in that** the oil represents from 10 to 40% by weight, based on the total weight of the composition.

12. The composition as claimed in any one of claims 1 to 11, **characterized in that** it is packaged in a jar.

13. A process for preparing the composition as claimed in any one of claims 1 to 11, **characterized in that** it comprises the following successive steps:

- mixing the vegetable butter, the wax and the oil at a temperature of 70 to 80°C,
- cooling the mixture to a temperature of 25 to 40°C,
- introducing air into said mixture until it reaches an expansion rate of 30 to 150%, wherein the expansion rate is defined and measured as described in the specification, and
- maturing the foam composition thus obtained.

14. The cosmetic use of the composition as claimed in any one of claims 1 to 11, to prevent and/or treat the signs of dry skin, in particular to nourish and/or soften the skin.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060147390 A
- US 8802118 B
- FR 2915892
- FR 2915877
- WO 2016150978 A